# FASCICULE DE BREVET EUROPEEN

(11) **EP 1 412 421 B1**
(45) Date de publication et mention de la délivrance du brevet: **07.03.2012**
(21) Numéro de dépôt: 01949519.1
(22) Date de dépôt: 25.06.2001
(51) Int. Cl.: C08J 9/26, A61L 27/56, C08L 23/20

(54) **POLYMERE BIOCOMPATIBLE A STRUCTURE TRIDIMENSIONNELLE A CELLULES COMMUNICANTES, PROCEDE DE PREPARATION ET APPLICATION EN MEDECINE ET EN CHIRURGIE**
BIOKOMPATIBLE POLYMERE MIT DREIDIMENSIONALER STRUKTUR UND MIT ZELLEN, VERFAHREN ZU DEREN HERSTELLUNG UND SEINE VERWENDUNG IN DER MEDIZIN UND IN DER CHIRURGIE
THREE-DIMENSIONAL STRUCTURE BIOCOMPATIBLE POLYMER WITH COMMUNICATING CELLS, PREPARATION METHOD AND USE IN MEDICINE AND SURGERY

(30) Priorité: 26.06.2000 FR 0008186
(43) Date de publication de la demande: 28.04.2004
(73) Titulaire: UNIVERSITÉ PIERRE ET MARIE CURIE (PARIS VI), 75252 Paris Cédex 05 (FR)
(72) Inventeur: HONIGER, Jiri, F-94800 Villejuif (FR); APOIL, André, F-78200 Mantes La Jolie (FR)
(74) Mandataire: Vaillant, Jeanne
(86) Numéro de dépôt international: PCT/FR2001/002007
(87) Numéro de publication internationale: WO 2002/000775

(56) Documents cités:
- EP-A- 0 286 220
- EP-A- 0 478 279
- US-A- 5 273 750
- US-A- 5 514 378
- US-A- 5 626 861

## Description

La présente invention concerne le domaine des bio-matériaux.

Elle concerne plus particulièrement un procédé de préparation d'un polymère poreux biocompatible à cavités communicantes de taille, de porosité, et de rigidité contrôlées. Elle concerne notamment l'application de ces matériaux biocompatibles à la culture de cellules *in vitro,* ainsi qu'à la préparation de supports biocompatibles ainsi ensemencés, tels quels ou encapsulés par un polymère ou son hydrogel semi-perméable et également biocompatible, comme implants dans différents organes ou tissus du corps humain ou animal, pour y remplacer de façon permanente ou temporaire un organe défaillant, et créer ainsi un organe bio-artificiel. Tel pourrait être le cas du pancréas bio-artificiel, du foie bio-artificiel, de la cornée bio-artificielle, du cartilage articulaire bio-artificiel, de l'os bio-artificiel, etc.

Elle concerne également l'application à la fabrication de supports des cellules transfectées, productrices d'un facteur de croissance tissulaire ou cellulaire, plus généralement d'une substance biologiquement active telle une cytokine, un facteur de croissance ou une molécule recombinante d'intérêt thérapeutique.

Cette matière poreuse à cavités communicantes peut également être implantée "nue" dans le corps vivant, pour combler le déficit de substances comme par exemple : la substance cartilagineuse en chirurgie maxillo-faciale ou la réalisation de prothèses mammaires.

Les bio-matériaux conformément à l'invention trouvent également leur application dans la préparation de filtres pour la bio-épuration de fluides biologiques, ou comme supports d'enzymes pour la réalisation d'un réacteur enzymatique.

Dans l'ensemble de ce texte, le terme de bio-matériaux doit être compris comme des matériaux non vivants utilisés dans un dispositif médical destiné à interagir avec les systèmes biologiques. Par définition, un bio-matériau est apte au contact avec des tissus ou des fluides vivants ou tissus du vivant. Ce contact, qui est évident dans le cas d'un implant, doit être étendu au contact qui se réalise à la surface ou à l'extérieur du corps humain ou animal, par exemple ceux qui se produisent avec le sang dans l'hémodialyse ou avec la cornée dans les lentilles de contact. Elle est également étendue aux matériaux utilisables en biotechnologie, notamment les matériaux pour la culture *in vitro* de cellules animales ou végétales. Un bio-matériau est par essence biocompatible. Par bio-compatibilité, on entend la capacité d'un matériau à être utilisé avec une réponse de l'hôte appropriée dans une application spécifique. Cette définition implique des propriétés "négatives" telles l'absence de toxicité, l'absence de réaction inflammatoire, l'absence d'activation du complément, l'absence de chute leucocytaire. Cela comprend également des "propriétés positives" qui impliquent que le matériau ne soit pas nécessairement le plus inerte possible mais au contraire fasse réagir le tissu vivant et contribue à l'activation métabolique des cellules qui lui sont en contact ou des tissus dans lesquels il est implanté ; c'est le cas notamment des matériaux ostéo-conducteurs qui facilitent la croissance osseuse.

Les bio-matériaux poreux de la présente invention entrent dans la catégorie des polymères ou des copolymères organiques. De fait, le procédé de l'invention et la matière à cavités communicantes obtenu par le procédé permettent à des cellules mises en culture de s'organiser dans ces cavités communicantes et le cas échéant d'y proliférer. Ces propriétés permettent non seulement la culture de cellules et la fabrication d'organes artificiels mais également la construction de tissus cellulaires transportables et transplantables, notamment dans la transplantation de tissus néo-cartilagineux, produits à partir de chondrocytes en culture.

Différents matériaux poreux à base de produits naturels ou synthétiques, organiques ou minéraux ont été décrits dans leur utilisation dans la culture de cellules *in vitro* et dans la transplantation de cellules vivantes. A titre d'exemple, il convient de signaler les utilisations et les développements de deux céramiques à base de phosphate de calcium : l'hydroxy apatite (HAP) et le phosphate tricalcique-β (TCB). L'hydroxy apatite phosphocalcique de formule Ca₁₀ (PO4)₆OH₂ est un matériau synthétique commercialisé comme substitut osseux synthétique par la société TECHNIMED. La difficulté avec ce type de matériau est d'arriver à synthétiser une HAP ayant juste la bonne taille de pores pour que la colonisation par des cellules, et notamment les cellules osseuses, se fasse correctement. Par ailleurs, l'utilisation de ces types de matériaux est limitée par les méconnaissances des mécanismes de dégradation, leur durabilité et leur résistance à la fracture, leur activité de surface et les possibilités de calcification.

De nombreux polymères naturels ou synthétiques ont également été décrits. A titre d'exemple, on peut citer des feutres en polyester ou en polytétrafluoroéthylène seul et traité avec un polyuréthanne (1) ; du polyéthylmétacrylate/tétrahydrofurfuryle-métacrylate (2) et (3), des éponges de collagène (4) ; du polyhydroxyéthylmétacrylate (5) ; des copolymères d'acides polyglycoliques et polylactiques (6).

Ont également été décrits des polyamides et/ou des polyaminoacides utilisables dans des formes retard de médicaments.

D'autres matériaux poreux ont été conçus pour la médecine ou la chirurgie.

Une méthode originale de réalisation d'une éponge en cellulose a été apportée en 1996 par O. Pajulo et coll. (9). Le principe de cette méthode est de coaguler la suspension contenant des cristaux et des dendrites du sel de Glauber et de la solution cellulosique. La taille des pores et l'épaisseur de la paroi entre les pores dépendent de la taille des cristaux et de leur quantité. Cette "éponge" a été ensuite implantée de façon sous-cutanée chez le rat, pour une étude de repousse cellulaire.

En 1997, Shapiro L. et Cohen S. (10) ont préparé une éponge rigide d'alginate, destinée à être ensemencée par des cellules, suivie d'une mise en culture et d'une transplantation dans le corps vivant.

US 5 514 378 A (CIMA LINDA G ET AL) concerne la préparation de membranes poreuses biocompatibles en dispersant des particules lixiviables dans une solution de polymère biocompatible. Le polymère biocompatible de la structure poreuse (membrane) n'est pas un hydrogel.

La majeure partie de ces matériaux constitue des matières biorésorbables, lesquelles disparaissent dans le temps plus ou moins long après leur implantation dans un organisme vivant, en laissant sur place des cellules et des tissus cellulaires.

Les principaux problèmes mal résolus avec l'utilisation de ces polymères ne concernent pas uniquement la bio-compatibilité à l'interface matériau/tissu. Pour les polymères non résorbables, on peut citer l'instabilité au rayonnement gamma ou la réactivité à certains types de médicaments ou à certains métaboliques. Il apparaît également extrêmement difficile d'éviter la variabilité constitutionnelle de chaque lot de fabrication. Il apparaît des risques de calcification, des risques liés aux additifs, aux composants de bas poids moléculaire, aux produits de dégradation *in vivo.*

Pour les polymères bio-résorbables, il manque cruellement de mesure de dégradation et de bio-résorption ainsi que des effets biologiques des produits de dégradation.

Globalement, l'utilisation de polymères ou de copolymères organiques ou minéraux, naturels et synthétiques est aujourd'hui encore mal contrôlée tant sur la reproductibilité de leur fabrication, de leur bio-compatibilité et des effets biologico-métaboliques sur les cellules ou les tissus avec lesquels ils sont en contact.

Le besoin existe toujours d'avoir un matériau biocompatible, apte à la croissance des cellules eucaryotes, et qui ne présente pas l'ensemble des inconvénients décrits ci-dessus. L'obtention d'un tel matériau passe par la mise en oeuvre d'un matériau déjà connu pour ses propriétés de bio-compatibilité et en particulier d'hémo-compatibilité, afin d'obtenir une structure apte aux différentes utilisations à la fois pour la culture de cellules *in vitro* que pour l'implantation dans un organisme vivant. Pour cette implantation tant comme organe artificiel que pour la régénération des tissus osseux ou cartilagineux, un procédé doit pouvoir être appliqué sur le bio-matériau utilisé afin d'en réaliser une structure en trois dimensions contenant de multiples cavités communiquant entre elles ainsi qu'avec la surface du corps au travers de celles se trouvant dans sa proximité ces cavités dont la taille et l'organisation peuvent être contrôlées permettant l'ensemencement, la croissance et le cas échéant la différenciation de cellules. Après colonisation de l'espace constitué par ces cavités, les cellules peuvent se différencier par l'action de facteurs de croissance ou de différenciation ajoutés ou produits par les tissus ou organes avec lesquels elles sont en contact.

Les différentes applications nécessitent également la mise en oeuvre d'un procédé permettant de maîtriser la taille des cavités, la forme et la rigidité (la plus ou moins grande souplesse) du polymère.

Selon l'invention, on peut réaliser ces objectifs avec un copolymère de la famille d'un polymère utilisé depuis de nombreuses années sous la forme de membranes pour l'hémodialyse ou sous sa forme hydrogel, pour des implants oculaires ou pour la préparation de pancréas artificiel (7). Ce copolymère a déjà été démontré comme biocompatible et hémo-compatible, et notamment quant à ses capacités de ne pas activer le système du complément (15), de ne pas induire de chute leucocytaire et de n'induire qu'une hypoxémie minimale (8). Le polymère en question est un copolymère appelé AN 69, fabriqué par la société HOSPAL R. & D. Int. (Meysieu, France).

Le procédé de l'invention utilise les propriétés mêmes de la fabrication de l'hydrogel illustrée dans le cas du copolymère d'acrylonitrile et de méthallylsulfonate de sodium, ladite fabrication comprenant successivement une étape solution et une étape gélification puis de formation d'un hydrogel. La formation et la définition des hydrogels est décrite par Honiger et al. (7). L'hydrogel se forme par précipitation d'une solution de polymère homogène. Dans un diagramme ternaire (polymère/solvant/ non-solvant), la courbe d'équilibre sépare une zone où tous les composants sont miscibles d'une autre zone ou deux phases se forment (une phase solide riche en polymère et une phase liquide pauvre ou appauvrie en polymère). Durant la formation de l'hydrogel, le système évolue depuis la solution initiale de polymère jusqu'à une composition où tout le solvant est remplacé par le non-solvant, ceci transformant le gel en hydrogel ; cet hydrogel ne comporte essentiellement plus que du non-solvant et du polymère. Cette succession d'étapes (forme liquide, forme gélifiée), le passage de la forme liquide à la forme gélifiée étant déclenché par le contact du copolymère avec un non solvant, permet d'envisager de réaliser cette gélification autour d'une matrice de forme et de porosité préalablement choisies en fonction de l'application ultérieure du copolymère biocompatible. Autrement dit, le concept à la base de l'invention est d'utiliser un moule ou une matrice qui confère au bio-matériau la porosité et la forme choisies, la rigidité étant déterminée par les conditions de la réalisation de l'hydrogel et notamment sa teneur en eau.

Le procédé réside sur une caractéristique essentielle du polymère qui est la capacité de passer d'un état liquide à un état non liquide, présentant une certaine rigidité. Ainsi, la présente invention s'applique par équivalence à tout polymère biocompatible pouvant, grâce à un facteur déclenchant, passer d'un état liquide à un état non liquide. Par état non liquide, on entend un état gélifié ou cristallisé ou pseudo-cristallisé, ou un état d'hydrogel.

Le choix du moule ou de la matrice utilisé pour conférer au bio-matériau sa forme et sa porosité est réalisé selon deux stratégies alternatives. La première est de choisir pour le moule ou la matrice un matériau dont la neutralité et la bio-compatibilité sont totales. Mais comme nous l'avons dit plus haut, aujourd'hui aucun matériau n'est connu qui présente à la fois les capacités d'avoir une porosité contrôlée, une bio-compatibilité et un contrôle de ces effets à long terme. La deuxième alternative est d'utiliser comme moule ou matrice n'importe quelle substance dont la taille et la porosité sont contrôlables et susceptibles, après la formation de l'hydrogel ou de la structure solide sur ledit moule, d'être éliminées. Cette élimination peut être réalisée par dissolution, ou par digestion enzymatique.

La solution de l'élimination totale du moule ou de la matrice avant utilisation *in vivo* du bio-matériau est préférée. Il est bien entendu que seul l'hydrogel ou le polymère rigidifié subsiste dans ce cas. Sa forme, sa porosité sont pré-déterminées par le moule ou la matrice, et sa rigidité par le contenu en eau ; l'ensemble des propriétés de bio-compatibilité déjà décrites depuis de nombreuses années et dans de nombreuses publications pour l'AN 69 et l'hydrogel d'AN 69 sont conservées dans les structures tridimensionnelles ainsi obtenues.

Ainsi, un premier mode de réalisation de l'invention porte sur un procédé d'obtention d'une structure tridimensionnelle poreuse à cavités communicantes constituée d'un polymère biocompatible comportant un état liquide et un état gélifié ou solide, comprenant les opérations suivantes:
- préparer un fritté de géométrie et de porosité choisies constitué d'une substance hydrosoluble ou hydrolysable, non soluble dans le solvant du polymère et soluble dans le non-solvant du polymère,
- préparer une solution comportant du polymère dans le solvant du polymère,
- imbiber ledit fritté avec une solution de polymère,
- mettre le fritté imbibé par la solution de polymère dans des conditions physiques de transformation du polymère biocompatible de l'état liquide en l'état gélifié ou solide, ou d'hydrogel incorporant ladite substance hydrosoluble ou hydrolysable,
- dissoudre ou hydrolyser, le cas échéant, le fritté par immersion du mélange dans un non-solvant du polymère,
- récupérer le polymère de géométrie et de porosité choisies sous forme gélifiée ou solide ou sous forme d'un hydrogel.

Dans un autre mode de réalisation de l'invention, l'invention porte sur un procédé d'obtention d'une structure tridimensionnelle poreuse à cavités communicantes constituée d'un polymère biocompatible comportant un état liquide et un état gélifié ou solide, comprenant les opérations suivantes :
- préparer une substance hydrosoluble ou hydrolysable, non soluble dans le solvant du polymère et soluble dans le non-solvant du polymère, sous forme de particules de taille et de géométrie choisies,
- préparer une solution comportant du polymère dans le solvant du polymère,
- réaliser un mélange hétérogène contenant une solution du polymère et de la substance hydrosoluble ou hydrolysable dans un moule,
- mettre le moule contenant le mélange hétérogène dans des conditions physiques de transformation du polymère biocompatible de l'état liquide en l'état gélifié ou solide,
- démouler le mélange ainsi gélifié ou solidifié ou sous forme d'hydrogel incorporant ladite substance hydrosoluble ou hydrolysable,
- dissoudre ou hydrolyser, le cas échéant, la substance hydrosoluble ou hydrolysable dans un non-solvant du polymère.
- récupérer le polymère de géométrie et de porosité choisies sous forme gélifiée ou solide ou sous forme d'un hydrogel.

Le moule destiné à donner sa forme et sa dimension à la structure tridimensionnelle peut être par exemple en élastomère de silicone.

Les deux modes de réalisation du procédé décrit ci-dessus reposent sur deux caractéristiques essentielles :
a) le polymère biocompatible utilisé doit avoir les propriétés de passer d'un état liquide à un état gélifié ou solide, cette transformation pouvant être maîtrisée par un déclencheur externe de type non-solvant du polymère, température, pH, par exemple, c'est-à-dire la propriété de former un hydrogel ;
b) l'utilisation d'une matrice qui assure le rôle d'un moule destiné à conférer la forme souhaitée au polymère biocompatible, ladite matrice ou ledit moule pouvant être éliminés, soit par dissolution, soit par hydrolyse.

Quand on parle de forme choisie, on entend la forme extérieure qui peut être choisie en fonction d'une géométrie souhaitée pour un implant. A titre d'exemple, la réalisation d'un implant pour une régénération osseuse doit avoir la géométrie souhaitée pour une implantation parfaite dans un site d'insertion. La forme peut être ainsi réalisée, soit en conférant dès le départ une forme à la matrice constituée de la substance hydrosoluble ou hydrolysable, ceci constituant le premier mode de réalisation du procédé, soit en préparant la substance hydrosoluble ou hydrolysable sous forme de billes ou microbilles dont la taille est choisie en fonction de la taille souhaitée pour les cavités communicantes ; ces billes ou ces microbilles sont alors mélangées avec la forme liquide du polymère biocompatible, ledit mélange étant préparé dans un moule de géométrie et de taille choisies. Après gélification et solidification du polymère, le moule est ensuite enlevé avant ou après dissolution ou hydrolyse de la substance.

Dans une forme préférée de l'invention, l'étape de gélification ou de solidification est réalisée par immersion dans un bain contenant un non-solvant du polymère. Comme cela apparaîtra dans les exemples, selon des procédés connus en eux-mêmes, le bain dit bain gélifiant ou solidifiant ou de formation d'hydrogel comporte de l'eau ou une solution aqueuse d'un sel biologiquement acceptable.

Les déposants ont trouvé que de manière surprenante certains matériaux, dans certaines conditions, ont des avantages considérable plus particulièrement dans le domaine des implants. Les bio-matériaux en question entrent dans la catégorie des hydrogels. Les hydrogels sont des réseaux hydrophiles tridimensionnels qui sont capables d'absorber de grandes quantités d'eau ou de fluide biologique et qui dans une certaine mesure ressemblent à un tissu biologique. Ils sont insolubles du fait de la présence d'un réseau de liens chimiques ou physiques, et peuvent être formés en réponse à un grand nombre de stimuli physiologiques ou physiques tels la température, la force ionique, le pH ou le contact avec des solvants.

Dans un mode préféré de réalisation de l'invention, les structures tridimensionnelles sont essentiellement à base d'hydrogel, c'est-à-dire que la structure est constituée d'un matériau homogène.

Dans le procédé selon l'invention, la solution de polymère comprend au moins :
- un polymère ou copolymère soluble dans les solvants aprotiques polaires inorganiques ou organiques ; et
- un solvant aprotique polaire organique ou inorganique du polymère ou copolymère, ce solvant étant de préférence compatible avec le non-solvant utilisé, c'est-à-dire miscible de préférence de 0 à 100%, avec le non-solvant.

Par solvant aprotique, on entend tout solvant qui n'échange pas de protons avec le milieu ambiant ou les substances dissoutes dans celui-ci.

Dans un mode de réalisation préféré de l'invention, un hydrogel préféré contient de 50 à 98 % d'eau. La capacité ionique de l'hydrogel peut être comprise entre 0 et environ 500 mEq/kg, de préférence 30 à 300 mEq/kg, de façon encore préférée entre 100 et 270 mEq/kg d'hydrogel. Des capacités ioniques basses (de l'ordre de 0) sont atteintes pour l'hydrogel d'homopolymère PAN (AN69 sans groupe méthallylsulfonate de sodium). De tels hydrogels peuvent être formés à partir d'une solution de polymères comprenant au moins :
- un copolymère d'acrylonitrile et d'un comonomère oléfinique insaturé porteur de groupements anioniques, ledit comonomère étant choisi dans le groupe constitué d'acide metallylsulfonique, d'acide metallylcarboxylique, d'acide metallylphosphorique, d'acide metallylphosphonique, d'acide metallylsulfirique éventuellement salifiés,
- un solvant aprotique polaire organique ou inorganique du copolymère.

Toutefois, il est possible que la solution de polymère dans le solvant comporte en outre du non-solvant du polymère.

Dans une réalisation encore préférée de l'invention, le copolymère est un copolymère d'acrylonitrile et de métallylesulfonate de sodium. Ce polymère a été décrit et utilisé comme matériau biocompatible dans de nombreuses applications. Ce polymère est l'AN 69 dont il est fait référence plus haut. Il s'agit d'un copolymère de polyacrylonitrile-méthallylsulfolnate de sodium d'un poids moléculaire d'environ 250.000. Son caractère anionique dépend du contenu du groupe sulfonique (3,3 mol %). Ce copolymère peut être dissous dans un solvant aprotique tel que le NN-diméthylformamide (DMF), le diméthylsulfoxyde (DMSO), le NN-diméthylacétamide (DMAA), ou le propylène carbonate (PC). A partir du copolymère polyacrylonitrile-sodium métallylesulfolnate, il est possible de former un hydrogel en précipitant une solution homogène dans un bain de précipitation (procédé par inversion de phases ou procédé par séparation de phases) par un procédé tel que décrit dans J. Honiger et al. (7).

Dans le procédé de l'invention, le polymère peut également être choisi dans un groupe contenant le polysulfone, le polyethersulfone, le polyhydroxy ethyl methacrylate, le polyhydroxy propyl methacrylate, ou leurs copolymères.

En fonction des applications, l'hydrogel peut contenir de 2 à 50 % de copolymères d'acrylonitrile et d'un comonomère non saturé portant des groupements anioniques, le ratio molaire acrylonitrile/co-monomère étant compris entre 90:10 et 100:0. Un solvant et un non solvant appropriés d'un tel copolymère, le rapport solvant/non solvant étant compris entre 500:1 et 0,5:1 en poids. Un tel hydrogel a une structure micro-poreuse et une capacité ionique comprise entre 0 et 500 mEq par kilo de gel, un contenu hydrique compris entre 50 et 98 %.

Ce polymère est utilisé depuis plus de vingt ans comme membrane de dialyse rénale sous forme de fibre creuse ou de feuille plane. Ses propriétés physiques et chimiques sont parfaitement connues, et il a fait la preuve depuis plus de vingt ans de son excellente bio-compatibilité avec le sang et le sérum. En particulier, dès 1978, il fut établi que la membrane d'AN 69 n'engendrait pas d'activation du complément donnant à une agrégation des leucocytes ni à une séquestration des agrégats formés dans la micro-circulation pulmonaire menant à son tour à une leucopénie et à un risque d'hypoxie (11).

Dans le procédé de l'invention, le solvant aprotique du copolymère sera de façon préférée, quand le polymère est un copolymère d'acrylonitrile et d'un comonomère de métallylesulfonate, choisi dans un groupe comprenant le NN-dimethylformamide (DMF), le dimethyl sulfoxide(DMSO), le NN-dimethylacetamide, le polypropylène carbonate, la N-methylpyrrolidone(NMP). Deux solvants préférés de par le recul et l'expérience accumulés dans son utilisation sont le DMSO et le DMF.

Les proportions respectives de chacun des éléments composant la solution de polymères peuvent varier selon les caractéristiques attendues pour le polymère biocompatible, notamment pour ce qui concerne sa rigidité. A titre d'exemple, une matière selon l'invention comportant 5 à 15 % de polymère donnera une éponge souple, déformable. Néanmoins, une matière contenant 25 à 35 % de polymère sera préférée et permet d'obtenir une substance poreuse de rigidité/souplesse contrôlées, en fonction du rapport en poids du polymère ou copolymère d'une part, et de la substance hydrosoluble ou hydrolysable, d'autre part.

Dans le procédé de l'invention, un fritté de géométrie ou de porosité préparé avec une substance hydrosoluble ou hydrolysable, ou cette même substance préparée sous forme de particules de taille et de géométrie choisies est imbibé ou mélangé au polymère biocompatible dans son état liquide.

De préférence, les procédés de l'invention sont mis en oeuvre essentiellement sans évaporation du solvant ou du non-solvant.

Dans une forme préférée de l'invention, la substance hydrosoluble ou hydrolysable non soluble dans le solvant du polymère et soluble dans le non-solvant du polymère est du saccharose aggloméré ou cristallisé.

Dans une forme encore préférée de l'invention, cette substance peut être un aggloméré de pseudo-cristaux de sucre de canne ou de betterave en morceaux ou en poudre. Les avantages de l'utilisation de cette substance sont sa parfaite tolérabilité en terme de toxicité, sa très bonne solubilité et enfin la facilité avec laquelle on peut moduler la forme et la taille des particules agglomérés.

Sur ce dernier point, l'utilisation de saccharose permet d'obtenir des particules de diamètre moyen compris entre 0,1 et 3 mm, conférant ainsi au polymère biocompatible des cavités communicantes de la taille choisie. Dans certains cas, après élimination du saccharose, un certain retrait des cavités peut se manifester. Celui-ci est homogène au sein de la mousse obtenue et reproductible pour un polymère ou copolymère donné. Ainsi, l'homme du métier pourra choisir la taille des particules en fonction de la taille des cavités communicantes recherchée.

Dans le procédé de l'invention, le non-solvant du polymère est une solution aqueuse d'un sel organique ou inorganique. De préférence, et dans le cas où la solution de polymères est composée de copolymères d'acrylonitriles et de métallylesulfonate de sodium, le solvant aprotique étant du DMSO, le non-solvant dudit polymère capable de former l'hydrogel est une solution de chlorure de sodium à 9 g par litre à la température ambiante d'environ 20° C.

Enfin, dans le procédé de l'invention, la substance hydrosoluble ou hydrolysable est , le cas échéant, éliminée par immersion dans de l'eau distillée à une température comprise entre 30 et 50° de préférence sous agitation. L'eau est renouvelée jusqu'à dissolution complète des cristaux de sucre, et libération des cavités communicantes. Le diamètre moyen des cavités peut être compris entre 0,1 et 3 mm.

Le diamètre des cavités dépend bien entendu de la taille des particules de la substance hydrosoluble ou hydrolysable qui sont éliminées, tout en pouvant être plus petit, à cause d'un effet de retrait constaté lors de la formation de l'hydrogel.

L'ensemble de ces opérations permet ainsi d'obtenir un polymère biocompatible de géométrie et de porosité choisies et utilisable dans de très nombreuses applications *in vitro*, *ex vivo* et *in vivo.*

La présente invention porte également sur une structure tridimensionnelle poreuse à cavités communicantes constituée d'au moins un polymère biocompatible, susceptible d'être obtenue par un procédé tel que décrit ci-dessus ; ces structures comportent de multiples cavités, lesquelles communiquent entre elles ainsi qu'avec la surface de ladite structure. Cette structure tridimensionnelle, à base d'hydrogel, poreuse à cellules communicantes peut être qualifiée de "mousse". Le terme de "mousse" qualifie tant l'existence de cavités communicantes entre elles et avec la surface de ladite mousse, que le fait de présenter des rigidités et des géométries variables.

Dans la suite du présent texte, le terme "mousse" ou "mousse de polymère" désignera toutes les structures tridimensionnelles susceptibles d'être obtenues par le procédé de l'invention.

De façon préférée, les mousses de polymère de l'invention seront des mousses d'hydrogel et de façon encore préférée des mousses d'AN 69 obtenues par le procédé.

Les mousses d'hydrogel selon l'invention, et plus particulièrement les mousses d'AN 69 peuvent comporter des résidus fonctionnalisés aptes à former des liaisons covalentes avec des résidus organiques. A titre d'exemple, ces résidus fonctionnalisés peuvent être des résidus -CHO, -NH₂, -COOH, -SH. Un exemple d'une telle fonctionnalisation est décrit dans la demande de brevet PCT/FR 98/00066 pour l'AN 69. Cet exemple n'est néanmoins pas limitatif dans la mesure où dans les demandes de brevets WO 92/07023 et WO 92/07006, il a été également décrit la fonctionnalisation d'autres polymères hydrophiles non chargés tels le polyéthylene glycol-hypoxy lié de façon covalente à une polyéthyleneimine.

L'avantage des mousses selon l'invention porteuses de résidus fonctionnalisés est la possibilité de coupler par liaison covalente ou ionique des ligands organiques ; à titre d'exemple, de tels ligands peuvent être sélectionnés dans un groupe comprenant des anticorps, des antigènes, des peptides, des protéines ou glycoprotéines, des hormones, des enzymes, des co-facteurs de ceux-ci, des substrats ou inhibiteurs de ceux-ci, des polysaccharides, des lectines, des toxines ou des anti-toxines, des acides nucléiques ou polynucléotides, des haptènes ou des ligands d'haptènes, des pigments ou des colorants. Il apparaît clairement à l'homme du métier que ce type de mousse fonctionnalisée sur laquelle des ligands de type cités ci-dessus peuvent être fixés présente un intérêt dès que l'on souhaite purifier, épurer, transformer, un composé, une substance ou un métabolite présent dans un fluide biologique ou dans un organe.

Ainsi, la présente invention porte sur l'utilisation de telles mousses fonctionnalisées comme modules pour la bio-épuration par affinité *in vitro*, *ex vivo* ou *in vivo* de molécules ou macromolécules biologiques.

La taille et la géométrie des cavités communicantes des mousses de biopolymère peuvent être choisies en fonction des cellules mises en culture et de leur organisation dans les cavités communicantes, plus particulièrement lorsque ces cellules se différencient au sein même de la mousse. Le domaine de cultures cellulaires connaît un essor important depuis déjà bien longtemps, et nombreux sont les appareils et les produits développés dans le but d'optimiser conditions vitales de cellules en culture. P. ex. Boite de Pétrie, étuve à CO₂, gamme de milieux nutritifs, fond de boîte traité avec des produits d'origine biologique, organique ou minérale, permettant aux cellules une meilleure organisation, adhérence, prolifération etc. lors de leur culture.

Néanmoins, la manipulation avec des cellules cultivées lors de leur implantation ou transplantation n'est pas facile. On doit d'abord les mettre en suspension pour effectuer l'opération de leur immunoprotection par micro ou macro-encapsulation mais les cellules sont déjà souvent organisées et adhérent sur la boîte, ce qui nécessite de les racler, avec un risque de les abîmer.

La mousse de biopolymère définie par la présente invention permet aux cellules mises en culture, de s'organiser dans ses cavités communicantes, de proliférer et de construire un tissus cellulaire transportable et transplantable avec ou sans immunoprotection comme ce sera expliqué dans des exemples, notamment dans la transplantation du tissu néo-cartilagineux, produit par des chondrocytes en culture.

Compte tenu du caractère hautement compatible du polymère des mousses de l'invention, celles-ci comporteront avantageusement des cellules animales ou végétales, dans un milieu approprié à leur prolifération et/ou à leur différenciation.

Une des premières applications de la présente invention est l'utilisation de ce type de mousse pour la culture de cellules animales ou végétales, le cas échéant recombinantes pour leur culture *in vitro* et la production de macromolécules biologiques d'intérêt.

Les mousses de polymères biocompatibles selon l'invention, et plus particulièrement les mousses d'hydrogel, et encore plus particulièrement les mousses d'AN 69 trouvent une application particulièrement avantageuse lorsqu'elles contiennent des cellules destinées à des implantations dans le corps humain ou animal. L'avantage de la structure des mousses à cavités communicantes est que lorsqu'elles sont ensemencées avec des cellules souches ou indifférenciées, il est possible de construire un tissu cellulaire à l'intérieur de cette mousse par pré-culture dans un milieu contenant les facteurs de croissance et/ou de différenciation appropriés.

Un autre type de mousses selon l'invention est porteur de chondrocytes ou des cellules stromales chondrogéniques. L'implantation de mousses porteuses de chondrocytes permet la réalisation d'un cartilage bio-artificiel ou le comblement d'un déficit osseux. Pour réaliser ces mousses porteuses de chondrocytes, un des moyens est de séparer les chondrocytes d'un cartilage articulaire prélevé dans une articulation d'un homme ou d'un animal, d'ensemencer les chondrocytes dans la mousse à cavités communicantes, de cultiver ces chondrocytes ensemencés dans le support immergé dans le milieu nutritif dans une étude à 37° sous atmosphère comportant 5 % des CO2, et de transplanter après culture la mousse porteuse des cellules ayant ainsi proliférées dans un cartilage articulaire chez un individu. Cette transplantation peut être autologue ou hétérologue, à savoir que les chondrocytes peuvent provenir d'un individu donneur présentant une compatibilité tissulaire avec le receveur (greffe allogénique), ou être prélevés sur un individu, cultivés et implantés sous forme de mousse porteuse des chondrocytes au niveau du cartilage ou de l'os à réparer du même individu (greffe autologue).

De la même façon, les mousses selon l'invention peuvent être ensemencées avec des cellules souches ou progénitrices d'une lignée cellulaire particulière. En effet, la moelle est composée de cellules hématopoïétiques en association étroite avec des cellules d'origine non hématopoïétiques et un support appelé le microenvironnement médullaire. Au sein de ce compartiment non hématopoïétique, se trouvent des cellules stromales qui sont des progéniteurs cellulaires possédant des caractéristiques multipotentes de différentiation vers les tissus connectiques spécifiques tels que l'os et le cartilage. Les cellules du stroma et de la moelle osseuse, qui représentent environ 3 % des cellules mono nucléées peuvent être isolées en incubant les cellules mononucléées avec des anticorps monoclonaux dirigés contre l'endogline (CD15) couplés à des billes magnétiques. Cet antigène se retrouve sur une population cellulaire hautement homogène ayant des capacités d'expansion et des propriétés chondrogéniques. La suspension cellulaire peut être ensuite isolée par tous moyens connus de l'homme du métier, dont un exemple peut être une colonne d'affinité attachée à un aimant afin de retenir les cellules positives qui seront collectées, analysées et mises en culture pour expansion. Cette mise en culture dans les mousses selon l'invention est réalisée en présence d'un milieu de culture en présence des facteurs de différenciation appropriés notamment le TGFβ3. La culture dans ces conditions permet ainsi d'obtenir un pseudo-tissu cellulaire implantable sur l'os ou sur le cartilage.

De la même façon, la présente invention porte sur une mousse de bio-polymères à cavités communicantes porteuses d'hépatocytes. Ces mousses peuvent être ensuite implantées, par exemple dans la cavité péritéonale. Cette transplantation d'hépatocytes syngéniques ou congéniques peut permettre de corriger à long terme des déficiences métaboliques sans encourir d'immunosuppression. Un tel exemple sur le potentiel thérapeutique de la transplantation d'hépatocytes peut être donné dans N. Gomez et al. (12). La transplantation des mousses de polymères biocompatibles, et plus particulièrement encore d'un hydrogel d'AN 69, porteuse d'hépatocytes permet d'augmenter la longévité et la tolérabilité du transplant.

Pour cette application, il est avantageux dans le cas d'une transplantation syngénique de protéger les cellules par un film ou une membrane semi-perméable d'immunoprotection. Un tel film, ou une telle membrane pourra avantageusement être un hydrogel de polymère ou de copolymère selon l'invention.

De la même façon, font également partie de l'invention des mousses de polymères selon l'invention porteuse d'îlots de Langherans. Les îlots de Langherans peuvent être obtenus par toute technique accessible à l'homme du métier au moment de sa mise en oeuvre. A titre d'exemple, on peut citer la technique décrite dans C. Delauney et al (13). Le transplant porteur des îlots de Langherans peut ainsi être assimilé à un pancréas bio-artificiel qui permet, après implantation, la production à long terme d'insuline et la régulation de la glycémie.

Dans une autre mode de réalisation de l'invention, les mousses de polymères biocompatibles, et plus particulièrement d'hydrogel 69, peuvent constituer des réacteurs cellulaires implantables in vivo pour la production de substances d'intérêt thérapeutique. L'implant porteur des cellules productrices peut être implanté, soit en sous-cutané, soit au niveau d'un organe ou d'un tissu particulier. A titre d'exemple, on peut ainsi traiter différentes pathologies chroniques avec des protéines thérapeutiques comme l'anémie avec l'erythropoïétine, l'hémophilie avec le facteur VIII ou le facteur IX, les déficits vasculaires avec les facteurs angiogéniques, ou les tumeurs solides avec les facteurs anti-angiogéniques. La faisabilité de telles techniques d'implantation a déjà été montrée par E. Payen et al. pour l'érythropoïétine (14). Un mini bio-réacteur selon l'invention peut également contenir des cellules productrices de vecteurs, de virus ou de plasmides recombinants pour la thérapie génique.

Le bio-réacteur peut être ainsi implanté *in situ* notamment en proximité des cellules que l'on souhaite traiter par cette méthode. A titre d'exemple, on peut citer l'implantation dans un tissu musculaire ou encore une implantation cérébrale pour respectivement le traitement de certaines maladies génétiques ou le traitement de certains cancers.

Un autre aspect de l'invention porte sur l'utilisation des mousses de polymères biocompatibles selon l'invention dans la fabrication d'une prothèse destinée à combler un déficit de substances dans un organe, notamment une prothèse mammaire ou le complément d'un tissu osseux. Dans cette utilisation, la mousse de polymère implantée ne comporte pas de cellules, mais comporte un milieu permettant à des cellules en contact avec ladite mousse de coloniser *in situ* cette dernière. Ainsi, après implantation, les cellules de l'organe dans lequel la mousse, porteuse d'un milieu de culture stérile approprié, est implantée peuvent proliférer et aider au comblement du déficit de l'organe.

Un autre aspect de l'invention est l'utilisation des mousses en polymères bio-compatibles selon l'invention à la fabrication de médicaments pour la libération contrôlée de principe actif. En effet, ces mousses d'hydrogel offrent un moyen particulièrement approprié pour administrer des principes actifs moléculaires ou macromoléculaires et en particulier des principes actifs de nature peptidique ou polypeptidique.

De manière générale, les mousses tridimensionnelles à cellules communicantes selon l'invention trouveront leur application à chaque fois que l'homme du métier souhaitera réaliser un implant avec des cellules indifférenciées ou différenciées d'un certain type donné. Les exemples ci-dessus ne sont pas limitatifs dans la mesure où de la même façon on peut envisager un implant porteur de cellules neuronales, de kératinocytes, etc.

Ces mousses trouveront également leur application à chaque fois que l'on voudra réaliser avant implantation une différenciation *in situ* de cellules souches, constituant la trame d'un tissu préformé qui pourra ensuite se greffer avantageusement sur un organe ou un tissu dont on souhaite combler une fonction déficiente.

Elles trouveront enfin leur application toutes les fois que l'utilisation de cellules en culture, gardant toutes leurs fonctions métaboliques, est recherchée comme par exemple, sans être limitant, les biosensors destinés par exemple à évaluer l'effet de certaines molécules ou effecteurs sur des cellules.

Une toute autre application de cette matière conformément à l'invention est basée sur l'écoulement des fluides et des gaz au travers de cette matière poreuse à cavités communicantes.
1) L'écoulement non laminaire des liquides dans les cavités communicantes de cette matière élimine la couche limite où le transfert s'effectue par diffusion et par conséquent augmente le contact direct entre le liquide et le polymère ou son hydrogel, qui constitue la matrice de ladite matière. Ceci pourrait être utilisé pour l'épuration des liquides, pour l'échange des ions des liquides, ainsi que pour la résorption des substances actives, déposées auparavant sur la surface des cavités, par des liquides ou des gaz passants. Il est bien entendu que les substances actives, déposées sur la surface des cavités, peuvent réagir seulement par contact avec les fluides ou des gaz passant et ainsi stimuler, inhiber ou accélérer les réactions se déroulant dans les fluides et les gaz (par exemple, ralentir la coagulation du sang, catalyser la synthèse des gaz, etc.)
2) Une autre application dans ce domaine est la possibilité d'effectuer un mélange parfait de plusieurs liquides ou des gaz au cours de leur passage au travers de cette matière.

Les exemples et photos ci-après, sans être limitatifs, permettent d'illustrer tant le procédé de réalisation de ces structures en polymère biocompatible tridimensionnelles que leur utilisation *in vitro* ou *in vivo.*

### LEGENDE DES FIGURES :

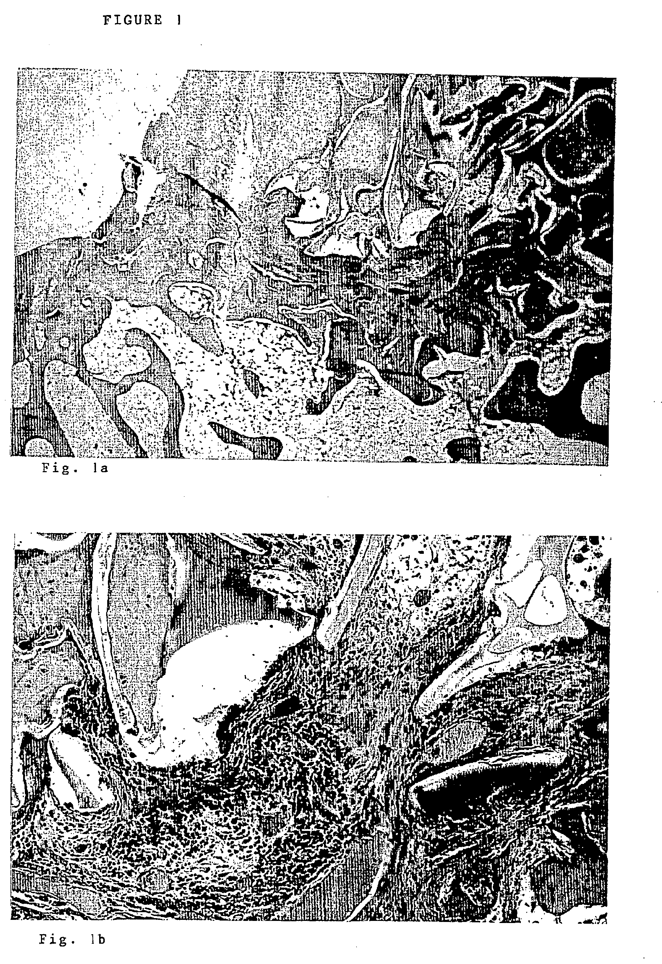

Figure 1 : photographie prise en microscopie optique d'une repousse osseuse basale et surface cartilagineuse articulaire dans la matière poreuse à structure tri-dimensionnelle préalablement ensemensée de chondrocytes de lapin et implantée sur le condyle fémoral après création d'un déficit cartilagineux. La figure 1a est une photo au grossissement de 15 fois et la figure 1 b est une photo au grossissement de 60 fois de la partie centrale de la photographie 1a montrant le détail de la repousse cartilagineuse.
Figuré 2 : photographie d'une mousse de polymère AN 69 obtenue par le procédé de l'exemple 4 ci-après. La photo du haut (2a) est au grossissement de x 17 et celle du bas (2b) est au grossissement de x 100.

### EXEMPLES :

### Exemple 1 : Réalisation d'une mousse souple à cavités communicantes, avant les dimensions approximatives de 0,8 cm x 1.3cm x 2,1 cm et la taille des pores de quelques dixièmes de millimètre

On prépare la solution polymère constituée de :
- 6 % de copolymère d'acrylonitrile et de méthallylsulfonate de sodium;
- 3 % de solution aqueuse de chlorure de sodium à 9 g/l ;
- 91 % de diméthylsulfoxide (DMSO)
par dissolution successive des composants sous agitation et à 50° C. Après le refroidissement à la température ambiante, on remplit un Bécher en polyméthylepentène (TPX) de 50 ml. On prend un morceau de sucre de canne 1,2 cm x 1,8 cm x 2,8 cm et à l'aide d'une pince on l'immerge très lentement et entièrement dans la solution du polymère. Dès l'échappement total d'air, retenu dans les interstices entre des cristaux du sucre, on enlève le morceau de sucre de la solution du polymère, on essuie l'excès de cette solution de toute la surface du morceau du sucre et on l'immerge pour quelques minutes dans un bain gélifiant, composé d'une solution aqueuse de chlorure de sodium à 9 g/l et ayant la température ambiante (20° C). Puis on le place dans de l'eau distillée, chaud (40°C), agitée et souvent renouvelée, pour que les cristaux du sucre se dissolvent et libèrent des cavités communicantes.

### Exemple 2 : Réalisation d'une mousse semi-riaide à cavités communicantes porteuse de chondrocytes

On prépare à ces fins un moule en élastomère de silicone, qui comporte un fond plat et lisse, bordé tout au tour par un bourrelet de 1,5 mm d'épaisseur.

Par la dissolution d'un copolymère d'acrylonitrile et de méthallylsulfonate de sodium dans du DMF, on prépare une solution du polymère à 25 %. On mélange ensuite cette solution en proportion de 1 : 4, avec les cristaux du sucre de canne, tamisés, de taille de 1-1,5 mm. On étale ce mélange à l'aide d'une spatule sur le moule en silicone, en respectant l'épaisseur donnée par la hauteur des bourrelets. On immerge ensuite tout dans un bain coagulant contenant du sérum physiologique. Après quelques minutes, on démoule la plaque formée et on procède à la dissolution des cristaux du sucre en la lavant avec de l'eau distillée à 40° C, comme décrit dans l'exemple 1.

La plaque en élastomère poreux est ensuite décontaminée par une solution contenant de l'acide peracétique (APA), soigneusement lavée avec le sérum physiologique stérile jusqu'à la disparition des dernières traces d'APA. Ensuite, elle est ensemencée par les chondrocytes isolés du cartilage articulaire du lapin, soit par leur injection à la seringue munie d'une équille, soit par "l'infiltration/aspiration" comme dans une éponge. Placée dans une boîte de Pétri contenant un liquide nutritif, la plaque en élastomère poreux à cellules communicantes ensemencée des chondrocytes est soumise à un procédé de culture cellulaire dans une étude à CO₂. Deux semaines plus tard, les chondrocytes s'organisent, prolifèrent et créent un pseudo tissu continu. Les résultats obtenus apparaissent sur la photographie de la figure 1.

### Exemple 3 : Réalisation d'une matière poreuse en faible épaisseur (0,5 mm), comme support pour les cellules vivantes, notamment pour des kératocytes

Le mélange homogène de la solution du polymère AN-69 à 25 % dans du dimethylacetamide (DMAA) et de six parties pondérales de cristaux de sucre de canne de taille de 0,1-0,5 mm est moulé par compression entre deux plaques de verre plat, ou aplati à l'aide d'un cylindre en verre, suivi d'une immersion de l'ensemble : moule/mélange dans un bain gélifiant et la dissolution des cristaux du sucre s'effectue par le même procédé que dans l'exemple 1.

### Exemple 4 : Réalisation d'un bloc-filtre en polymère à cellules communicantes, pouvant être utilisé pour divers traitements des liquides circulants dans des pores au-travers de ce bloc-filtre

On prépare alors une solution contenant 25 % de copolymère d'acrylonitrile et de méthallylsulfonate de sodium et 75 % de Diméthylformamide. Dans un Bécher en verre ou en matière plastique résistante au Diméthylformamide, on mélange à la spatule 9 % de cette solution avec 91 % de sucre de canne cristallisé. On procède ensuite au transfert de ce mélange dans un autre Bécher en verre ou en matière plastique résistant au Diméthylformamide, et on tasse ce mélange à l'aide d'une spatule courbée et/ou à l'aide d'un cylindre ou d'autre Bécher, ayant un diamètre légèrement inférieur, pour qu'il puisse fonctionner comme un piston de tassement. On immerge le Becher avec le mélange tassé dans de l'eau distillée ou dans une solution aqueuse composée de divers sels minéraux ou organiques de préférence biologiquement acceptables. Après quelques minutes, on démoule le bloc-filtre ainsi formé, et on laisse dissoudre les cristaux de sucre par un lavage en continu ou en discontinu avec de l'eau distillée ou avec des solutions aqueuses des divers sels.

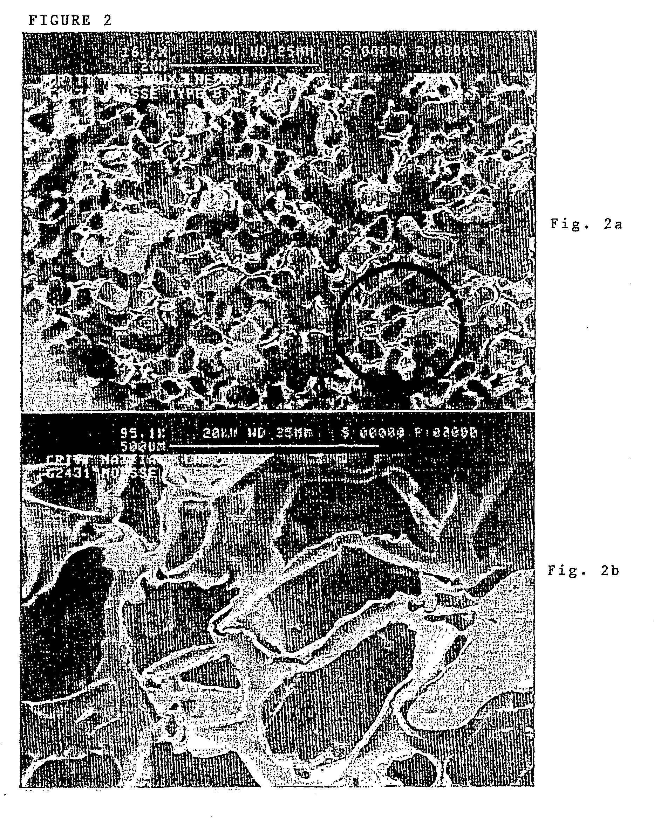

On obtient ainsi une matière poreuse à cavités communicantes en hydrogel AN-69, ayant dans les cavités une contenance en eau de 78 % (en poids) et la teneur en eau dans l'hydrogel est environ de 75 % (en poids) (Figure 2).

### Exemple 5 : Obtention d'une forme de polymère à cellules communicantes par moulage du mélange de la solution polymérique et du sucre cristallisé

On prépare à ce fait un mélange identique comme décrit dans l'exemple 4. Ce mélange est ensuite introduit à la spatule et tassé à l'aide d'une baguette en polytetrafluoréthylène dans un tube en verre. On introduit ensuite de l'eau distillée dans l'intérieur du tube et on fait sortir par gravité ou par faible courant d'eau un cylindre, lequel après la dissolution totale de sucre cristallisé devient un cylindre en polymère à cellules communicantes.

### Exemple 6 : Réalisation d'un bloc polymère AN 69 contenant dans des cellules communicantes jusqu'à 97 % d'eau et pouvant servir après des examens nécessaires comme un implant de comblement de la substance manquante (implant mammaire par exemple)

On prépare un mélange contenant 3,5 % de la solution polymérique (25 % copolymère d'acrylonitrile et de méthallylsulfonate de sodium et 75 % de Diméthylformamide) et 96,5 % du sucre cristallisé. On transmet ce mélange dans un moule, on le tasse, puis on immerge le moule rempli dans de l'eau ou dans des solutions aqueuses des divers sels. Après la dissolution totale du sucre, on obtient donc un bloc d'élastomère poreux, contenant près de 97 % d'eau dans ses cavités.

### Exemple 7 : Réalisation d'une matière poreuse à cavités communicantes en Polyethersulfone

Le mélange homogène de la solution du Polyethersulfone à 25 % dans du DMF et de dix parties pondérales de sucre de canne en cristaux de taille de 0,5 - 1 mm est moulé, coagulé avec la dissolution du sucre exactement selon le procédé décrit dans l'exemple 4.

### Exemple 8 : Réalisation d'une matière poreuse à cavités communicantes en Poyhydroxypropyl méthacrylate

Le mélange homogène de la solution du Polyhydroxypropyl méthacrylate à 60 % dans du DMF et de dix parties pondérales de sucre de canne en cristaux de taille de 0,5 - 1 mm est moulé, coagulé avec la dissolution du sucre exactement selon le procédé décrit dans l'exemple 4. On obtient ainsi une matière souple avec une grande plasticité, poreuse à cavités communicantes en Polyhydroxypropyl méthacrylate.

### Exemple 9 : Réalisation d'une matière souple et poreuse à cavités communicantes espacées en Polysulfone

Le mélange homogène de la solution du Polysulfone à 20 % dans du DMF et de dix parties pondérales de sucre de canne en cristaux de taille de 0,5 - 1 mm est moulé, coagulé avec la dissolution du sucre exactement selon le procédé décrit dans l'exemple 4.

### Exemple 10 : Réalisation d'une matière rigide et poreuse à cavités communicantes serrées en Polysulfone

Le mélange homogène de la solution du Polysulfone à 20 % dans du DMF et de cinq parties pondérales de sucre de canne en cristaux de taille de 0,5 - 1 mm est moulé, coagulé avec la dissolution du sucre exactement selon le procédé décrit dans l'exemple 4.

On voit ainsi que, par modification du rapport entre le polysulfone/DMF et le sucre de canne qui est de 1/10 dans l'exemple 9 et de 1/5 dans l'exemple 10, on obtient des mousses de qualité radicalement différentes, à savoir souples et à cavités espacées dans le premier cas, et rigides et à cavités serrées dans le deuxième cas.

### REFERENCES BIBLIOGRAPHIQUES

(1) Messner K: Lohmander L.-S., Gillquist J., "Neocartilage after artificial cartilage repair in the rabbit: histology and proteoglycan fragments in joint fluid", J. Biomed. Mat. Res. (1993) 27: 949-954.
(2) Reissis N., Kayser M., Bentley G., Downes S., "A hydrophilic polymer system enhanced articular cartilage régénération in vivo", J. Mater. Sci.: Mat. in Medicine (1995) 6: 768-772.
(3) Sawtell R., Downes S., Kayser M., "An in vitro investigation of the PEMA/THFMA system using chondrocyte culture", J. Mater. Sci.: Mat. in Medicine (1995) 6: 676-679.
(4) Toshia Fujisato, Toshinobu Sajiki, Quiang Liu, Yoshito Ikada, "Effect of basic fibroblast growth factor on cartilage régénération in chondrocyte-seeded collagen sponge scaffold", Biomaterials (1996) 17: 155-162.
(5) Reginato A., lozzo R., Jimenez S., "Formation of nodular structures resembling mature articular cartilage in long-term primary cultures of human fetal epiphyseal chondrocytes on a hydrogel substrate", Arthritis & Reumatism (1994) 37: 1338-1349.
(6) Sittinger M., Reitzel D., Dauner M., Hierlemann H., Hammer C., Kastenbauer E., Planck H., Burmester G., Bujia J., "Resorbable polyesters in cartilage engineering: Affinity and biocompatibility of polymer fiber structures to chondrocytes" J. Biomed. Mat. Res. (1996) 33: 57-63.
(7) Honiger J., Darquy S., Reach G., Muscat E., Thomas M., Collier C., "Preliminary report on cell encapsulation in a hydrogel made of biocompatible material, AN 69, for the development of a bioartificial pancreas", The International Journal of Artificial Organs (1994), vol. 17, 1: 046-052.
(8) Honiger J., Balladur P., Mariani P., Calmus Y., Vaubourdolle M., Delelo R., Capeau J. et Nordlinger B., "Permeability and biocompatibility of a new hydrogel used for encapsulation of hepatocytes", Biomaterials (1995), 16: 753-759.
(9) Pajulo O., Viljanto J., Loenberg B., Hurme T., Loenquist K., Saukko P., "Viscose cellulose sponge as an implantable matrix: Changes in the structure increase the production of granulation tissue", J. Biomed Mat. Res. (1996) 32: 439-446.
(10) Shapiro L., Cohen S., "Novel alginate sponges for cell culture and transplantation", Biomaterials (1997) 18: 583-590.
(11) Jacob A.I., Gavellas G., Zarco R., Perez G., Bourgoignie J.-J., leukopenia, hypoxia and complement functions with a different hemodialysis membranes kidney Int. (1980) 18: 505-509.
(12) Gomez N., Balladur P., Calmus Y., Baudrimont M., Honiger J., Delelo R., Myara A., Crema E., Trivin F., Capeau J. et Nordlinger B., "Evidence for survival and metabolic activity of encapsulated xenogeneic hepatocytes transplanted without immunosuppression in gunn rats", Transplantation (June 27, 1997), vol. 63, 12: 1718-1723.
(13) Delaunay C., Honiger J., Darquy S., Capron F., Reach G., "Bioartificial pancreas containing porcine islets of langerhans implanted in low-dose streptozotocin-induced diabetic mice: effect of encapsulation medium", Diabetes & Metabolism (1997), 23: 219-227.
(14) Payen E. Dalle, B., Honiger J., Henri A., Kuzniak L., Rouyer-Fessard P., Benzard Y., "Régulation de la production d'érythropoïétine transgénique in vivo", Congrès de European Hemotologic Applications (E.H.A.) of Barcelona (June 1999).
(15) Honiger J., Couturier C., Goldschmidt P., Maillet F., Kazatchkine M.-D., Laroche L., "A new anionic hydrogel for korneal surgery", J. Biomed. Mat. Res. (1997) 37: 548-553.

## Revendications

1. Procédé d'obtention d'une structure tridimensionnelle poreuse à cavités communicantes constituée d'au moins un polymère biocompatible comportant un état liquide et un état gélifié ou solide, le polymère biocompatible étant un hydrogel, comprenant les opérations suivantes :
- préparer un fritté de géométrie et de porosité choisies constitué d'une substance hydrosoluble ou hydrolysable, non soluble dans le solvant du polymère et soluble dans le non-solvant du polymère ;
- préparer une solution comportant du polymère dans le solvant du polymère ;
- imbiber ledit fritté avec la solution de polymère ;
- mettre le fritté imbibé par la solution de polymère dans des conditions physiques de transformation du polymère biocompatible de l'état liquide en l'état gélifié ou solide ou d'hydrogel incorporant ladite substance hydrosoluble ou hydrolysable ;
- dissoudre ou hydrolyser , le cas échéant, le fritté par immersion du mélange dans un non-solvant du polymère ;
- récupérer le polymère de géométrie et de porosité choisies sous forme d'un hydrogel.

2. Procédé d'obtention d'une structure tridimensionnelle poreuse à cavités communicantes constituée d'au moins un polymère biocompatible comportant un état liquide et un état gélifié ou solide, le polymère biocompatible étant un hydrogel, comprenant les opérations suivantes :
- préparer une substance hydrosoluble ou hydrolysable, non soluble dans le solvant du polymère et soluble dans le non-solvant du polymère, sous forme de particules de taille et de géométrie choisies ;
- préparer une solution comportant du polymère dans le solvant du polymère ;
- réaliser un mélange hétérogène contenant une solution du polymère et de la substance hydrosoluble ou hydrolysable dans un moule ;
- mettre le moule contenant le mélange hétérogène dans des conditions physiques de transformation du polymère biocompatible de l'état liquide en l'état gélifié ou solide, ou d'hydrogel ;
- démouler le mélange ainsi gélifié ou solidifié ou d'hydrogel incorporant ladite substance hydrosoluble ou hydrolysable ;
- dissoudre ou hydrolyser , le cas échéant, la substance hydrosoluble ou hydrolysable dans un non solvant du polymère ;
- récupérer le polymère de géométrie et de porosité choisies sous forme d'un hydrogel.

3. Procédé selon la revendication 1 ou 2 dans lequel l'étape de gélification ou de solidification, ou de formation de l'hydrogel est réalisée par immersion dans un bain contenant un non-solvant du polymère.

4. Procédé selon la revendication 1 à 3 dans lequel la substance hydrosoluble ou hydrolysable non soluble dans le solvant du polymère et soluble dans le non-solvant du polymère est du saccharose aggloméré ou cristallisé.

5. Procédé selon la revendication 1,2 ou 3 dans lequel le polymère biocompatible est un hydrogel contenant 50 à 98% d'eau, et une capacité ionique comprise entre 30 et 300 mEq/kg.

6. Procédé selon la revendication 5, **caractérisé en ce que** l'hydrogel a une capacité ionique comprise entre 100 et 270 mEq/kg.

7. Procédé selon l'une des revendications 1 à 6 dans lequel la solution de polymère comprend au moins :
- un polymère ou copolymère soluble dans les solvants aprotiques polaires inorganiques ou organiques,
- un solvant aprotique polaire organique ou inorganique du copolymère.

8. Procédé selon l'une des revendications 1 à 7, dans lequel la solution de polymère comporte en outre du non-solvant du polymère.

9. Procédé selon l'une quelconque des revendications 1 à 5, **caractérisé en ce que** le polymère est soluble dans les solvants aprotiques miscibles au non-solvant.

10. Procédé selon l'une des revendications 7 à 9 dans lequel la solution de polymère comprend au moins un copolymère d'acrylonitrile et d'un comonomère oléfinique insaturé porteur de groupements anioniques, ledit comonomère étant choisi dans le groupe constitué d'acide metallylsulfonique, d'acide metallylcarboxylique, d'acide metallylphosphorique, d'acide metallylphosphonique, d'acide metallylsulfirique éventuellement salifiés.

11. Procédé selon la revendication 10 dans lequel le copolymère est un copolymère d'acrylonitrile et de metallylsulfonate de sodium, ou AN 69.

12. Procédé selon l'une des revendications 7 à 10 dans lequel le polymère est choisi dans un groupe contenant le polysulfone, le polyéthersulfone, le polyhydroxy éthyl méthacrylate, le polyhydroxy propyl méthacrylate ou leurs copolymères.

13. Procédé selon l'une des revendications 1 à 12 dans lequel le solvant du polymère est un solvant aprotique choisi dans un groupe comprenant le NN-diméthyl formamide (DMF), le diméthyl sulfoxide (DMSO), le NN-diméthylacétamide, la N-méthylpyrrolidone (NMP).

14. Procédé selon l'une des revendications 1 à 13, **caractérisé en ce que** le procédé est mis en oeuvre essentiellement sans évaporation du solvant ou du non-solvant.

15. Structure tridimensionnelle poreuse à cellules communicantes constituée d'au moins un polymère biocompatible et comportant de multiples cavités, lesquelles communiquent entre elles ainsi qu'avec la surface de ladite structure, ledit polymère comportant un état liquide et un état gélifié ou solide et étant sous forme d'un hydrogel.

16. Structure selon la revendication 15 **caractérisée en ce que** l'hydrogel est un hydrogel de copolymère d'acrylonitrile et de metallylsulfonate de sodium, ou AN 69.

17. Structure selon la revendication 15 ou 16 **caractérisée en ce que** le diamètre moyen des cavités est compris entre 0,1 et 3 mm.

18. Structure selon l'une des revendication 15 à 17 **caractérisée en ce que** qu'elle contient des chondrocytes ou des cellules stromales chondrogéniques dans un milieu approprié à leur prolifération et/ou à leur différenciation.

19. Structure selon l'une des revendications 15 à 17 **caractérisée en ce que** qu'elle contient des ilôts de Langherans dans un milieu approprié à leur survie.

20. Structure selon l'une des revendications 15 à 17 **caractérisée en ce que** qu'elle contient des cellules animales, le cas échéant recombinées et productrices de substances d'intérêt thérapeutique, dans un milieu de culture approprié à leur survie et à leur sécrétion.

21. Structure selon l'une des revendications 15 à 17 **caractérisée en ce que** le polymère biocompatible comporte des résidus fonctionnalisés aptes à former des liaisons covalentes avec des résidus organiques, notamment -CHO, -NH2, -COOH, -SH.

22. Utilisation d'une structure tridimensionnelle poreuse à cellules communicantes selon la revendication 18 pour la fabrication d'un cartilage bio-artificiel ou un comblement d'un déficit osseux.

23. Utilisation d'une structure tridimensionnelle poreuse à cellules communicantes selon la revendication 19 pour la fabrication d'un pancreas bio-artificiel.

24. Utilisation d'une structure tridimensionnelle poreuse à cellules communicantes selon la revendication 20 à la fabrication d'un réacteur implantable pour la production de substances thérapeutiques in vivo.

25. Utilisation selon la revendication 24 dans laquelle la substance est une molécule d'intérêt thérapeutique.

26. Utilisation selon la revendication 24 dans laquelle la substance est un vecteur ou un virus recombinant porteur d'un gène d'intérêt pour la thérapie génique.

27. Utilisation d'une structure tridimensionnelle poreuse à cellules communicantes selon la revendication 21 sur laquelle des ligands ont été greffés le cas échéant par liaison covalente comme module pour la bioépuration par affinité ex vivo ou in vivo de molécules biologiques.

28. Utilisation d'une structure tridimensionnelle poreuse à cellules communicantes selon la revendication 15 ou 16 dans la fabrication d'une prothèse destinée à combler un déficit de substance dans un organe, notamment une prothèse mammaire ou le comblement d'un tissu cartilagineux.

29. Utilisation d'une structure tridimensionnelle poreuse à cellules communicantes selon la revendication 15 pour la fabrication d'une forme à libération contrôlée de principes actifs de médicament.

## Claims

1. A process for producing a porous three-dimensional structure with communicating cavities constituted by at least one biocompatible polymer comprising a liquid state and a gelled or solid state, the biocompatible polymer being a hydrogel, comprising the following operations:
• preparing a frit with a pre-selected geometry and porosity constituted by a hydrosoluble or hydrolyzable substance which is not soluble in the polymer solvent and which is soluble in the polymer non-solvent;
• preparing a solution comprising a polymer in the polymer solvent;
• impregnating said frit with the polymer solution;
• placing the frit impregnated with the polymer solution under physical conditions for transforming the biocompatible polymer from the liquid state into the gelled or solid state, or a hydrogel incorporating said hydrosoluble or hydrolyzable substance;
• dissolving or hydrolyzing the frit, as appropriate, by immersing the mixture into a polymer non-solvent;
• recovering the polymer with the selected geometry and porosity in the form of a hydrogel.

2. A process for producing a porous three-dimensional structure with communicating cavities constituted by at least one biocompatible polymer comprising a liquid state and a gelled or solid state, the biocompatible polymer being a hydrogel, comprising the following operations:
• preparing a hydrosoluble or hydrolyzable substance which is not soluble in the polymer solvent and which is soluble in the polymer non-solvent, in the form of particles with a selected size and geometry;
• preparing a solution comprising the polymer in the polymer solvent;
• forming a heterogeneous mixture containing a solution of the polymer and a hydrosoluble or hydrolyzable substance in a mould;
• placing the mould containing the heterogeneous mixture under physical conditions for transforming the biocompatible polymer from the liquid state into the gelled or solid state or hydrogel;
• unmoulding the gelled or solidified of hydrogel mixture incorporating said hydrosoluble or hydrolyzable substance;
• as appropriate, dissolving or hydrolyzing the hydrosoluble or hydrolyzable substance in a polymer non-solvent;
• recovering the polymer with the selected geometry and porosity in the gelled or solid form or in the form of a hydrogel.

3. A process according to claim 1 or claim 2, in which the gelling or solidification or hydrogel formation step is carried out by immersion in a bath containing a polymer non-solvent.

4. A process according to claims 1 to 3, in which the hydrosoluble or hydrolyzable substance that is not soluble in the polymer solvent and soluble in the polymer non-solvent is agglomerated or crystalline saccharose.

5. A process according to claim 1, 2 or 3, in which the biocompatible polymer is a hydrogel containing 50% to 98% of water and with an ionic strength in the range 30 to 300 mEq/kg.

6. A process according to claim 5, **characterized in that** the hydrogel has an ionic strength in the range 100 to 270 mEq/kg.

7. A process according to one of claims 1 to 6, in which the polymer solution comprises at least:
• a polymer or copolymer that is soluble in inorganic or organic polar aprotic solvents;
• an organic or inorganic polar aprotic solvent for the copolymer.

8. A process according to one of claims 1 to 7, in which the polymer solution additionally comprises a polymer non-solvent.

9. A process according to any one of claims 1 to 5, **characterized in that** the polymer is soluble in aprotic solvents that are miscible with the non-solvent.

10. A process according to one of claims 7 to 9, in which the polymer solution comprises at least one copolymer of acrylonitrile and an unsaturated olefinic co-monomer carrying anionic groups, said co-monomer being selected from the group formed by methallylsulphonic acid, methallylcarboxylic acid, methallylphosphoric acid, methallylphosphonic acid and methallylsulphuric acid, optionally in their salt forms.

11. A process according to claim 10, in which the copolymer is a copolymer of acrylonitrile and sodium methallylsulphonate, or AN69.

12. A process according to one of claims 7 to 10, in which the polymer is selected from the group formed by polysulphone, polyethersulphone, polyhydroxyethylmethacrylate, polyhydroxypropylmethacrylate, or copolymers thereof.

13. A process according to one of claims 1 to 12, in which the polymer solvent is an aprotic solvent selected from the group formed by N,N-dimethylformamide (DMF), dimethylsulphoxide (DMSO), N,N-dimethylacetamide and N-methylpyrrolidone (NMP).

14. A process according to one of claims 1 to 13, **characterized in that** the process is carried out essentially without evaporating off the solvent or non-solvent.

15. A porous three-dimensional structure with communicating cells constituted by at least one biocompatible polymer and comprising multiple cavities, which communicate with each other and with the surface of said structure, said polymer comprising a liquid state and a gelled or solid state and being in the form of a hydrogel.

16. A structure according to claim 15, **characterized in that** the hydrogel is a hydrogel of a copolymer of acrylonitrile and sodium methallylsulphonate, or AN69.

17. A structure according to claim 15 or claim 16, **characterized in that** the mean diameter of the cavities is in the range 0.1 to 3 mm.

18. A structure according to one of claims 15 to 17, **characterized in that** it contains chondrocytes or chondrogenic stromal cells in a medium appropriate for their proliferation and/or differentiation.

19. A structure according to one of claims 15 to 17, **characterized in that** it contains islets of Langerhans in a medium appropriate for their survival.

20. A structure according to one of claims 15 to 17, **characterized in that** it contains animal cells, recombinant if appropriate, and which produce substances of therapeutic interest, in a culture medium appropriate for their survival and to their secretion.

21. A structure according to one of claims 15 to 17, **characterized in that** the biocompatible polymer comprises functionalized residues that can form covalent bonds with organic residues, in particular -CHO, -NH₂, -COOH and -SH.

22. Use of a three-dimensional porous structure with communicating cells according to claim 18, for the production of a bio-artificial cartilage or to replenish a bone deficit.

23. Use of a three-dimensional porous structure with communicating cells according to claim 19, for the production of a bio-artificial pancreas.

24. Use of a three-dimensional porous structure with communicating cells according to claim 20, for the production of an implantable reactor for *in vivo* production of therapeutic substances.

25. Use according to claim 24, in which the substance is a molecule of therapeutic interest.

26. Use according to claim 24, in which the substance is a recombinant virus or vector carrying a gene of interest, for gene therapy.

27. Use of a three-dimensional porous structure with communicating cells according to claim 21, onto which ligands have been grafted, if appropriate by covalent bonds, as a module for *ex vivo* or *in vivo* affinity biopurification of biological molecules.

28. Use of a three-dimensional porous structure with communicating cells according to claim 15 or claim 16, in the production of a prosthesis intended to overcome a substance deficit in an organ, in particular a mammary prosthesis or for replacing cartilage tissue.

29. Use of a three-dimensional porous structure with communicating cells according to claim 15, for the production of a form for controlled release of the active principles of drugs.

## Patentansprüche

1. Verfahren zur Gewinnung einer porösen dreidimensionalen Struktur mit kommunizierenden Hohlräumen, bestehend aus wenigstens einem biokompatiblen Polymer, das einen flüssigen Zustand und einen gelierten oder festen Zustand aufweist, wobei das biokompatible Polymer ein Hydrogel ist, das die folgenden Operationen enthält:
- die Zubereitung eines Sinterkörpers mit ausgewählten Geometrie und Porosität, bestehend aus einer wasserlöslichen oder hydrolysierbaren Substanz, die im Lösungsmittel des Polymers unlöslich und im Nichtlösungsmittel des Polymers löslich ist;
- die Zubereitung einer Lösung, die Polymer im Lösungsmittel des Polymers enthält;
- die Anfeuchtung dieses Sinterkörpers mit der Polymerlösung;
- die derartige physikalische Zubereitung des mit dem Polymerlösung angefeuchteten Sinterkörpers, so dass das biokompatible Polymer von einem flüssigen Zustand in einen gelierten oder festen Zustand verwandelt werden kann, oder sogar in ein Hydrogel, das diese wasserlösliche oder hydrolysierbare Substanz integriert;
- das Lösen oder Hydrolysieren, gegebenenfalls, des Sinterkörpers, wobei die Mischung in ein Nichtlösungsmittel des Polymers getaucht wird;
- das Sammeln des Polymers mit ausgewählten Geometrie und Porosität in der Form eines Hydrogels.

2. Verfahren zur Gewinnung einer porösen dreidimensionalen Struktur mit kommunizierenden Hohlräumen, wobei diese Struktur wenigstens aus einem biokompatiblen Polymer besteht, das einen flüssigen Zustand und einen gelierten oder festen Zustand aufweist, wobei das biokompatible Polymer ein Hydrogel ist, das die folgenden Operationen enthält:
- die Zubereitung einer wasserlöslichen oder hydrolysierbaren Substanz, die im Lösungsmittel des Polymers unlöslich und im Nichtlösungsmittel des Polymers löslich ist, in Form von Partikeln mit ausgewählten Grösse und Geometrie;
- die Zubereitung einer Lösung, die Polymer im Lösungsmittel des Polymers enthält;
- das Ausführen einer heterogenen Mischung, die eine Lösung des Polymers und der wasserlöslichen oder hydrolysierbar Substanz in einer Giessform enthält;
- die derartige physikalische Zubereitung der die heterogene Mischung enthaltenden Giessform, so dass das biokompatible Polymer von einem flüssigen Zustand in einen gelierten oder festen Zustand oder in eine Hydrogel verwandelt werden kann;
- die Entnahme der so gelierten oder festen Mischung oder der diese wasserlösliche oder hydrolysierbare Substanz enthaltenden Hydrogel-Mischung aus der Giessform;
- das Lösen oder Hydrolysieren, gegebenenfalls, der wasserlöslichen oder hydrolysierbaren Substanz in einem Nichtlösungsmittel des Polymers;
- das Sammeln des Polymers mit ausgewählten Geometrie und Porosität in der Form eines Hydrogels.

3. Verfahren nach Anspruch 1 oder 2, in welchem die Hydrogel-Gelierungs-, Erstarrungs- oder Bildungsstufe durch Eintauchen in ein ein Nichtlösungsmittel des Polymers enthaltendes Bad ausgefürht wird.

4. Verfahren nach Anspruch 1 bis 3, wobei die wasserlösliche oder hydrolysierbare Substanz, die im Lösungsmittel des Polymers unlöslich und im Nichtlösungsmittel des Polymers löslich ist, agglomerierte oder kristallisierte Saccharose ist.

5. Verfahren nach Anspruch 1, 2 oder 3, in welchem das biokompatible Polymer ein 50-98%-wasserhaltiges Hydrogel ist und eine ionische Kapazität zwischen 30 und 300 mEq/kg aufweist.

6. Verfahren nach Anspruch 5, **dadurch gekennzeichnet, dass** das Hydrogel eine ionische Kapazität zwischen 100 und 270 mEq/kg aufweist.

7. Verfahren gemäß einem der Ansprüche 1 bis 6, in welchem die Polymerlösung mindestens die folgenden Komponenten umfasst:
- ein in den inorganischen oder organischen aprotisch-polaren Lösungsmitteln lösliches Polymer oder Copolymer,
- ein organisches oder inorganisches aprotisch-polares Lösungsmittel des Copolymers.

8. Verfahren gemäß einem der Ansprüche 1 bis 7, in welchem die Polymerlösung sogar Nichtlösungsmittel des Polymers ferner enthält.

9. Verfahren nach einem der Ansprüche 1 bis 5, **dadurch gekennzeichnet, dass** das Polymer in den aprotischen, mit dem Nichtlösungsmittel mischbaren Lösungsmitteln löslich ist.

10. Verfahren gemäß einem der Ansprüche 7 bis 9, in welchem die Polymerlösung mindestens ein Copolymer aus Acrynitril und einem anionische Gruppen tragenden, ungesättigten olefinischen Comonomer umfasst, wobei dieses Comonomer gewählt ist aus der Gruppe bestehend aus eventuell versalzenen Metallylsulfonsäure, Metallylcarboxylsäure, Metallylphosphor säure, Metallylphosphonsäure, Metallylschwefelsäure.

11. Verfahren nach Anspruch 10, in welchem das Copolymer ein Copolymer aus Acrylnitril und Sodiummetallylsulfonat beziehungsweise AN 69 ist.

12. Verfahren nach einem der Ansprüche 7 bis 10, in welchem das Polymer gewählt ist aus einer gruppe bestehend aus Polysulfon, Polyethersulfon, Polyhydroxyethylmethacrylat, Polyhydroxypropylmethacrylat oder ihren Copolymeren.

13. Verfahren nach einem der Ansprüche 1 bis 12, in welchem das Lösungsmittel des Polymers ein aprotisches Lösungsmittel ist, gewählt aus einer gruppe bestehend aus NN-dimethylformamid (DMF), Dimethylsulfoxid (DMSO), NN-dimethylacetamid, N-methylpyrrolidon (NMP).

14. Verfahren nach einem der Ansprüche 1 bis 13, **dadurch gekennzeichnet, dass** das Verfahren im Wesentlichen ohne Verdampfung des Lösungsmittels oder des Nichtlösungsmittels ausgeführt wird.

15. Poröse dreidimensionale Struktur mit kommunizierenden Zellen, bestehend aus wenigstens einem biokompatiblen Polymer und umfassend zahlreiche Hohlräume, die miteinander sowohl als mit der Oberfläche dieser Struktur kommunizieren, wobei dieses Polymer einen flüssigen Zustand und einen gelierten oder festen Zustand aufweist und in der Form eines Hydrogels ist.

16. Struktur nach Anspruch 15, **dadurch gekennzeichnet, dass** das Hydrogel aus einem Copolymer aus Acrylnitril und Sodiummetallylsulfonat beziehungsweise AN 69 besteht.

17. Struktur nach Anspruch 15 oder 16, **dadurch gekennzeichnet, dass** der Durchschnittsdurchmesser der Hohlräume zwischen 0,1 und 3 mm beträgt.

18. Struktur nach einem der Ansprüche 15 bis 17, **dadurch gekennzeichnet, dass** sie in einem Medium, das zu deren Wucherung und/oder Differenzierung geeignet ist, Chondrocyten oder chondrogenische stromale Zellen enthält.

19. Struktur nach einem der Ansprüche 15 bis 17, **dadurch gekennzeichnet, dass** sie in einem zum Überleben geeigneten Medium Langerhans-Inseln enthält.

20. Struktur nach einem der Ansprüche 15 bis 17, **dadurch gekennzeichnet, dass** sie Tierzellen enthält, die gegebenenfalls rekombiniert sind oder Substanzen mit therapeutischem Interesse herstellen können. und zwar in einem zum Uberleben und zur Sekretion geeigneten Kulturmedium.

21. Struktur nach einem der Ansprüche 15 bis 17, **dadurch gekennzeichnet, dass** das biokompatible Polymer funktionalisierte Restgehalte enthält, die kovalente Bindungen mit organischen Restgehalten, insbesondere -CHO, -NH2, -COOH, -SH bilden können.

22. Verwendung einer porösen dreidimensionalen Struktur mit kommunizierenden Zellen nach Anspruch 18, zur Herstellung eines bioartifiziellen Knorpels oder zur Füllung eines Knochendefizites.

23. Verwendung einer porösen dreidimensionalen Struktur mit kommunizierenden Zellen nach Anspruch 19, zur Herstellung eines bioartifiziellen Pankreas.

24. Verwendung einer porösen dreidimensionalen Struktur mit kommunizierenden Zellen nach Anspruch 20, zur Bildung eines implantierbaren Reaktors zur Herstellung von in vivo therapeutischen Substanzen.

25. Verwendung nach Anspruch 24, in welcher die Substanz eine Moleküle mit therapeutischem Interesse ist.

26. Verwendung nach Anspruch 24, in welcher die Substanz, ein für die Gentherapie interessantes Gen tragendes rekombinantes Vektor oder Virus ist.

27. Verwendung einer porösen dreidimensionalen Struktur mit kommunizierenden Zellen nach Anspruch 21, auf welcher Liganden gegebenenfalls durch eine kovalente Bindung als ein Modul zur Bioreinigung durch ex vivo oder in vivo Verwandtschaft von biologischen Molekülen verpflanzt worden sind.

28. Verwendung einer porösen dreidimensionalen Struktur mit kommunizierenden Zellen nach Anspruch 15 oder 16, zur Herstellung einer Prothese, die zur Fülllung eines Substanzdefizites in einem Organ, insbesondere einer Brustprothese oder zur Füllung eines Knorpelgewebes ausgelegt ist.

29. Verwendung einer porösen dreidimensionalen Struktur mit kommunizierenden Zellen nach Anspruch 15, zur Herstellung einer Art von kontrollierter Freigabe von Arzneimittelwirkstoffen.
